# EUROPEAN PATENT SPECIFICATION

(11) **EP 4 401 885 B1**
(45) Date of publication and mention of the grant of the patent: **12.08.2026**
(21) Application number: 22870664.4
(22) Date of filing: 15.09.2022
(51) Int. Cl.: B01L 9/06, B01L 3/00, A61J 1/16, B01L 9/00, B65D 25/10, B65D 65/46, A61J 7/00, B65D 71/70

(54) **SUSTAINABLE AND RECYCLABLE PULP TRAY FOR BLOOD COLLECTION TUBES**
NACHHALTIGE UND WIEDERVERWERTBARE ZELLSTOFFSCHALE FÜR BLUTENTNAHMERÖHRCHEN
PLATEAU DE PÂTE DURABLE ET RECYCLABLE POUR TUBES DE COLLECTE DE SANG

(30) Priority: 15.09.2021 US 202163244408 P
(43) Date of publication of application: 24.07.2024
(73) Proprietor: Becton, Dickinson and Company, Franklin Lakes, NJ 07417 (US)
(72) Inventor: LI, Lingyu, Berwyn, Pennsylvania 19312 (US); CHANG, Yu-Wen, Ridgewood, New Jersey 07450 (US); DESAI, Niket, Sanjaybhai, Branchburg, New Jersey 08876 (US); ROWEK, Nicholas, Wayne, New Jersey 07470 (US); SNEIDER, Mark, Sumter, South Carolina 29150 (US)
(74) Representative: dompatent
(86) International application number: PCT/US2022/043595
(87) International publication number: WO 2023/043879

(56) References cited:
- CN-A- 104 495 014
- US-A- 3 305 438
- US-A- 4 722 440
- US-A- 5 788 929
- US-A1- 2009 065 458
- US-A1- 2009 065 458
- US-A1- 2010 192 813
- US-A1- 2015 238 263
- US-A1- 2015 238 263
- US-A1- 2019 343 721

## Description

### CROSS REFERENCE TO RELATED APPLICATION

The present application claims priority to United States Provisional Application Serial No. 63/244,408, filed September 15, 2021.

### BACKGROUND OF THE INVENTION

### Field of the Invention

The present invention relates to a tray for holding, transporting, and storing biological collection containers, and more specifically, a tray of molded plant-based pulp or recycled plant-based pulp for holding, transporting, and storing blood collection containers.

### Description of Related Art

Medical biological fluid collection containers, such as blood collection tubes, are typically cylindrical in shape with a semispherical bottom portion. Generally, blood collection tubes are stored and transported in trays. The trays are produced with expanded polystyrene (EPS) or other plastic materials, which are rarely recycled for logistic and economic reasons and, therefore, are treated as waste that is placed in landfills. EPS and other plastic trays are also bulky and use a substantial amount of materials.

There is, thus, a need for a more recyclable and sustainable tray for storing and transporting blood collection containers that can be manufactured economically.

US 2009/065458 A1 discloses a rack for centrifuge tubes including a plurality of receptacles sized to snugly receive one of a variety of different diameter centrifuge tubes. The rack may be formed of a variety of biodegradable, compostable materials, including for example molded polypropylene or organic resins such as a corn-based resin or potato-based resin. The rack further includes a lattice of recesses defined within an upper surface of the rack, which recesses extend between and connect the receptacles to add rigidity to the rack and flexibility to the individual receptacles.

US 2010/192813 A1 discloses a tube loading system suitable for rapidly loading, handing, manipulation and storing large numbers of tubes.

US 2015/238263 A1 discloses components packaging structure for pharmaceutical containers, comprising at least one tray, which has at least one open side for introducing and extracting at least one supporting shelf which is parallel to a bottom plane of the tray and which rests in correspondence of the inner walls of the tray; said supporting shelf has a spatially predetermined distribution of first seats or cavities within which closing components and/or generic accessories of said containers, such as rubber caps, nuts with crimping snap or other types of sealing nuts and/or caps, are placed, so that the vertical axis of each closure component is perpendicular to said bottom plane of the tray.

### SUMMARY OF THE INVENTION

The present invention is directed to a tray for storing biological fluid collection containers according to claim 1 comprising a body comprising an upper surface comprising a perimeter portion having an outer perimeter and an inner perimeter, and a recess extending downwardly from and surrounded by the inner perimeter of the perimeter portion. The recess is divided into a plurality of wells, each well adapted to receive at least a portion of a biological fluid collection container, and the tray comprises molded plant-based pulp.

Inner sidewalls of the plurality of wells adjacent to the perimeter portion may be defined by a body inner sidewall extending downwardly from the inner perimeter of the perimeter portion, a longitudinal partition extending in a longitudinal direction from a first side of the body inner sidewall to an opposing second side of the body inner sidewall, and a plurality of webs extending in a transverse direction between the body inner sidewall and the partition. Inner sidewalls of the plurality of wells that are not adj acent to the perimeter portion are defined by two longitudinal partitions extending in a longitudinal direction from a first side of the body inner sidewall extending downwardly from the inner perimeter of the perimeter portion to an opposing second side of the body inner sidewall, and a plurality of webs extending in a transverse direction between the partitions. The plurality of wells may have a closed bottom surface.

At least a portion of the inner sidewalls of the plurality of wells may be discontinuous such that an opening is provided between adjacent wells. The plurality of wells may be defined by a body inner sidewall extending downwardly from the outer perimeter of the perimeter portion, a longitudinal partition extending in a longitudinal direction from a first side of the body inner sidewall to an opposing second side of the body inner sidewall, and a plurality of webs extending in a transverse direction between the body inner sidewall and the partition or by two longitudinal partition extending in a longitudinal direction from a first side of a body inner sidewall extending downwardly from the inner perimeter of the perimeter portion to an opposing second side of the body inner sidewall, and a plurality of webs extending in a transverse direction between the partitions, and the opening may be defined by the webs.

The plurality of wells may be arranged in rows extending in the longitudinal direction of the tray. The position in the longitudinal direction of ends of a first row may be offset from the position in a longitudinal direction of ends of a second row adjacent in the transverse direction to one side of the first row and/or offset from the position in the longitudinal direction of ends of a third row adjacent in the transverse direction to an opposite side of the first row.

The inner sidewalls of the plurality of wells may have a shape substantially corresponding a shape of the biological fluid collection container that is to be received by the tray, the closed bottom surfaces of the plurality of wells have a shape substantially corresponding a shape of a bottom surface of the biological fluid collection container that is to be received by the tray, and/or the plurality of wells may be adapted to hold cylindrical biological fluid collection containers.

The tray may further comprise an outer sidewall extending downwardly from the outer perimeter of the perimeter portion. The outer sidewall may be substantially perpendicular to the upper surface or may be angled with respect to the upper surface. The outer sidewall may include a plurality of ribs. The ribs may be embossed in the outer sidewall such that the ribs are recesses on an outer surface of the outer sidewall and protrusions on an inner surface of the outer sidewall or the ribs are protrusions on an outer surface of the outer sidewall and recesses on an inner surface of the outer sidewall.

According to the present invention, the tray further comprises a base comprising a closed bottom end, an open top end, and a sidewall extending therebetween, the closed bottom end and sidewall of the base defining a compartment that receives the body. The base may further comprise a flange extending laterally outward from an upper perimeter of the sidewall of the base, and at least a portion of the perimeter portion of the body may be supported by the flange when the body is received in the compartment of the base. According to the present invention, the closed bottom end of the base includes at least one recess that receives a bottom end of one of the plurality of wells. Both the body and the base comprise molded plant-based pulp.

The present invention is also directed to a tray for storing biological fluid collection containers comprising a container body comprising a plurality of interconnected wells, each well adapted to receive at least a portion of a biological fluid collection container, and a base comprising a closed bottom end, an open top end, and a sidewall extending therebetween, the closed bottom end and sidewall of the base defining a compartment adapted to receive the container body. The container body and the base comprises molded plant-based pulp.

The plurality of wells may be arranged in rows extending in a longitudinal direction of the container body, and the position in the longitudinal direction of ends of a first row are offset from the position in a longitudinal direction of ends of a second row adjacent in a transverse direction to one side of the first row and/or offset from the position in a longitudinal direction of ends of a third row adjacent in the transverse direction to an opposite side of the first row. The plurality of wells may be arranged in rows extending in a longitudinal direction of the container body and webs may connect sidewalls of each well to sidewalls of all adjacent wells in the same row and in adjacent rows in the transverse direction. The webs may extend along a portion of the height of the sidewalls of the wells or along the entire height of the sidewalls of the wells. At least one of the webs attached to the sidewall of at least a portion of the plurality of wells may include an opening that extends partially or fully along the height of the sidewall. Every other web in a row may contain an opening and no more than one web connected to any well may have an opening.

The plurality of wells may have an open bottom surface or a closed bottom surface.

Inner sidewalls of the plurality of wells may have a shape substantially corresponding a shape of the biological fluid collection container that is to be received by the tray and/or the closed bottom surfaces of the plurality of wells may have a shape substantially corresponding a shape of a bottom of the biological fluid collection container that is to be received by the tray.

The container body may comprise molded plastic

The present invention is also directed to a method for manufacturing the tray comprising molding the tray from plant-based pulp or recycled plant-based pulp by thermoforming or injection molding moistened plant-based pulp or recycled plant-based pulp.

### BRIEF DESCRIPTION OF THE DRAWINGS

FIG. 1 is a front side perspective view of a tray according to a first embodiment of the invention;
FIG. 2 is a top view of the tray of FIG. 1;
FIG. 3 is an expanded view of area A of FIG. 2;
FIG. 4 is a side view of the tray of FIG. 1;
FIG. 5 is an expanded front side perspective view of a tray according to a second embodiment the invention;
FIG. 6 is another expanded front side perspective view of the tray FIG. 5;
FIG. 7 is an expanded front side perspective view of a tray according to a third embodiment the invention;
FIG. 8 is a top view of the tray of FIG. 7;
FIG. 9 is an expanded front side perspective view of the tray of FIG. 7;
FIG. 10 is a front side perspective view of the tray of FIG. 7;
FIG. 11 is an expanded front side perspective view of a tray not being part of the present invention;
FIG. 12 is a cross-sectional view of the tray of FIG. 11;
FIG. 13 is a front side perspective view of a tray not being part of the present invention;
FIG. 14 is a cross-sectional view of the tray of FIG. 11 and/or the tray of FIG. 13, not being part of the present invention;
FIG. 15 is an expanded front side perspective view of a tray not being part of the present invention;
FIG. 16 is an expanded front side perspective view of a tray not being part of the present invention; and
FIG. 17 is a cross-sectional perspective view of a portion of the tray of FIG. 16, with a fluid collection container retained therein.

### DESCRIPTION OF THE INVENTION

For purposes of the description hereinafter, spatial orientation terms, as used, shall relate to the referenced embodiment as it is oriented in the accompanying drawings, figures, or otherwise described in the following detailed description. However, it is to be understood that the embodiments described hereinafter may assume many alternative variations and configurations. It is also to be understood that the specific components, devices, features, and operational sequences illustrated in the accompanying drawings, figures, or otherwise described herein are simply exemplary and should not be considered as limiting. "Including", "such as", "for example" and like terms means "including/such as/for example but not limited to".

The present application is directed to a recyclable and sustainable tray for the storage and transport of biological fluid collection containers.

The tray may be made from plant-based pulp or recycled plant-based pulp molded into a three-dimensional (3D) shape. The pulp may come from naturally grown plants, for example, bamboo, sugarcane also known as bagasse, eucalyptus, and wood fibers including poplar wood fibers. Other non-conventional materials are also explored at both industrial and laboratory scales, such as potato, starch, coconut husk and natural latex. Alternatively, the pulp may be recycled pulp coming from cardboard, corrugate box, de-inked newspaper, or any other suitable clean paper source.

The tray may be produced by thermoforming or injection molding. When produced by thermoforming, the raw material pulp is soaked in water and mixed to achieve a consistent slurry. Additives, such as hydrophobic additives or fillers, may be included in the slurry to provide the finished tray with moisture resistance and/or strength. For example, Alkyl ketene dimers (AKDs) can be added to pulp slurry to improve water/moisture resistance and achieve higher mechanical strength. Typically AKDs are in the form of aqueous solution containing up to 30% solid AKDs. In one example, Dymer VP 902/17 is added to pulp slurry in the range of 0.05- 5wt% of total dry pulp amount. In another example, Dymer VP994/5 HS is added to pulp slurry in the range of 0.05- 5wt% of total dry pulp amount. Fillers such as bauxite, kaolin, chalk, talc, titanium dioxide, calcium sulfate, aluminum oxide, etc. can be added in the range of 2-25 wt% to slurry as well for mechanical strength. In our internal evaluation of moisture resistance, the prototype pulp tray can sustain iodine solution without obvious signs of absorbing iodine or leak through up to 2 hours. In terms of its mechanical strength to top loading application requirement, in a two tray sets tested separated at tube distance in carton under load of 110N equivalent to full load experienced by carton, trays displayed less than 0.5mm displacement. In another example, 27N which equals to 50% of total load experienced by the carton and the set of pulp tray is applied to each face of the top edge of each tray, trays displayed less than 0.05mm displacement. A metal net supported by a perforated mold and having the shape of the finished tray is lowered into the slurry and the water is pulled from the moistened pulp through the metal mesh by applying a vacuum. The wet part is moved to a heated mold where it is compressed between two matched halves of a mold having the shape of the finished tray and completely dried. During pressing and drying, the surface of the tray becomes smooth and good dimensional accuracy can be achieved. The pressing may also improve the mechanical properties of the tray.

When produced by injection molding, which is widely used in plastic part manufacturing, a mixture including the pulp, hydrophobic additive, fillers, and water is injected into a mold having the shape of the finished tray. In the mold, the moisture is removed from the mixture. Removal of the moisture may be achieved by frequently opening the mold slightly during the drying process.

Any additives used in the thermoforming process, or the injection molding process may be food safe, i.e., suitable for human consumption, and chosen for minimal impact on the environment. Common disposal options for end of life of pulp trays are recycling with paper product and biodegradation/ composting. Additives used are preferred suitable for biodegradation and recycling.

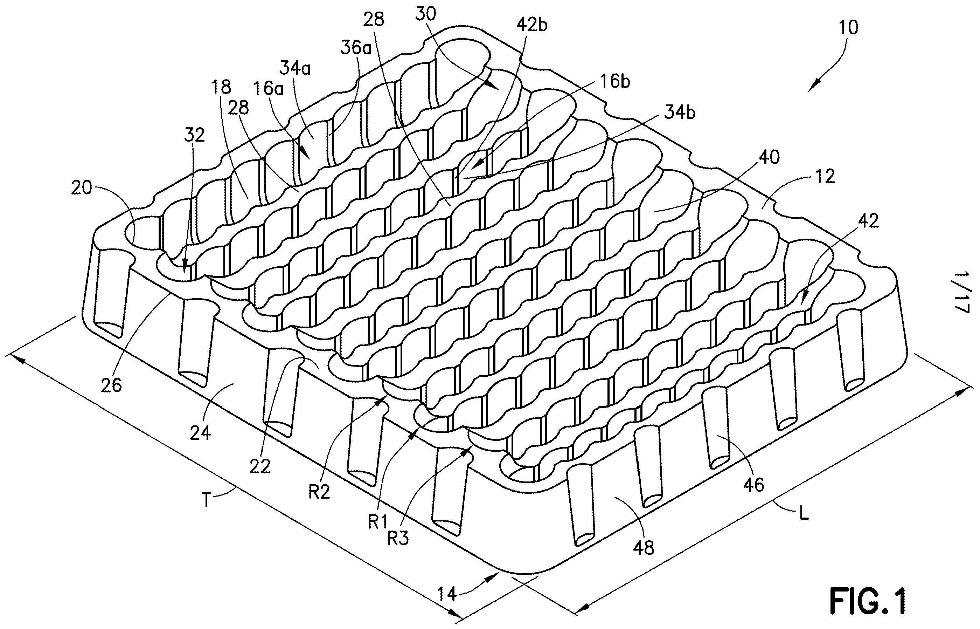

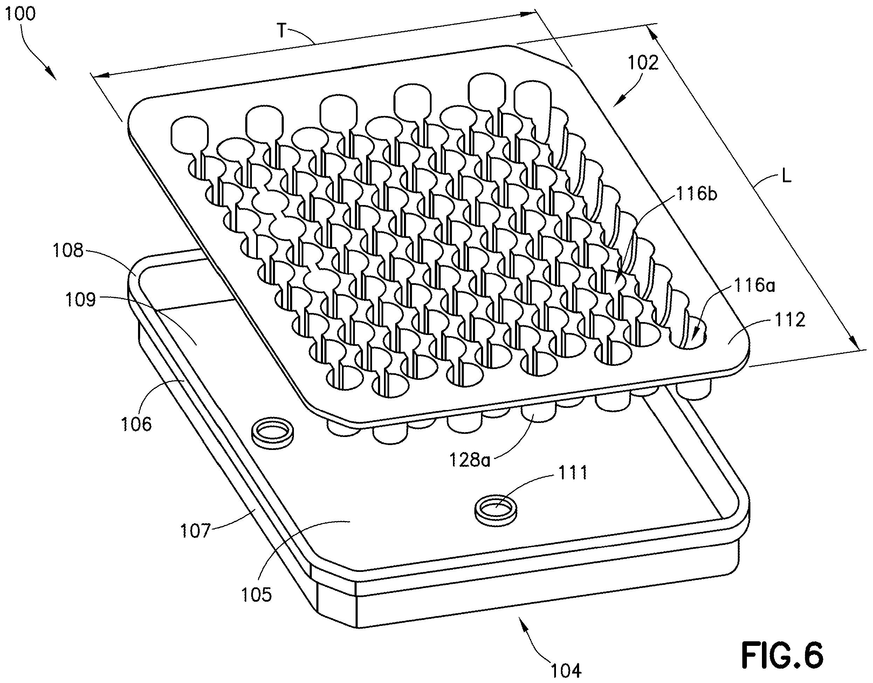

The tray may also be produced by using pulp slurry, known as thermal forming process or completely dry fibers, such as dry-molded fiber technology, or a combination of both processes. While thermal forming and post press process can produce parts of more complex geometry design with condensed cavities such as (but not limited to) a one piece tray, as illustrated in FIG. 1, or an inner tray in a two piece design, as illustrated in FIG. 6, dry molded fiber technology can deliver many other functionalities such as ( but not limited to) tighter dimension tolerance, high throughput, surface featuring (printing, embossing, debossing, coloring, patterning), zero draft angle, and/or under-cuts and multi-compartment. It is specifically noted that a tighter dimension tolerance/resolution, such as 0.75-1 mm over the typical tray length can be observed by thermal forming, as compared with <0.5 mm by dry fiber molding. Dry molded fiber technology uses pulp or paper as material source and converts them into cellulose fiber web, using air, for dry molding. The process offers high throughput speed and no water is needed. It is contemplated herein that a two-piece design tray can be made with a combination of thermal forming the inner portion, and dry pump molding the outer or bottom portion.

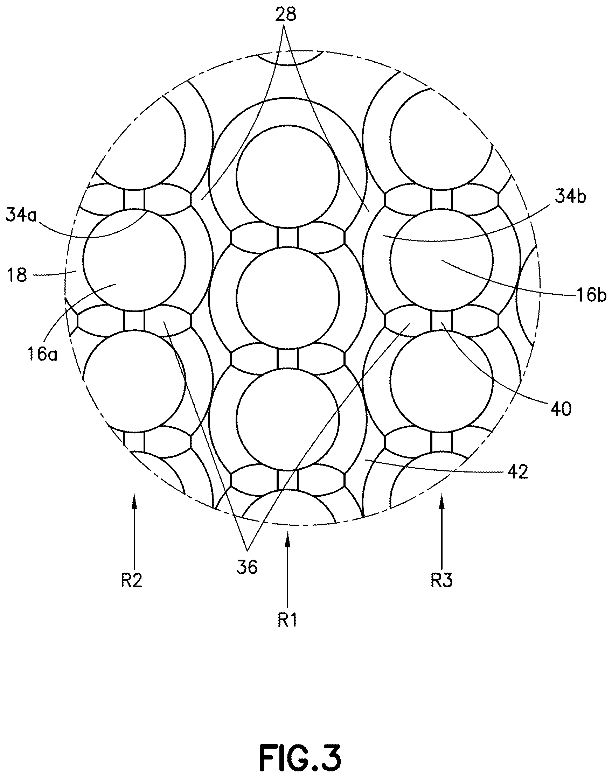

As shown in FIGS. 1-3, the tray 10 comprises an upper surface 12, a lower surface 14, a plurality of wells 16a, 16b extending from the upper surface 12 to the lower surface 14, an inner sidewall 18 extending downwardly from an inner perimeter 20 of a perimeter portion 22 of the upper surface 12, and an outer sidewall 24 extending downwardly from an outer perimeter 26 of the perimeter portion 22 of the upper surface 12. The tray 10 may have a substantially rectangular or square overall shape.

The perimeter portion 22 of the upper surface 12 has an inner perimeter 20 and an outer perimeter 26. The inner sidewall 18 extends downwardly from the inner perimeter 20 and at least partially defines the sidewalls 34a of a portion of the plurality of wells 16a.

At least a portion of the sidewalls 34a of the plurality of wells 16a adjacent to the perimeter portion 22 of the upper surface 12 are defined by the inner sidewall 18, a longitudinal partition 28 extending in the longitudinal direction L from a first side 30 of the inner sidewall 18 to an opposing second side 32 of the inner sidewall 18, and a plurality of webs 36a extending in the transverse direction T between inner sidewall 18 and the partition 28.

The inner sidewall 34b of the plurality of wells 16b that are not adjacent to the perimeter portion 22 of the upper surface 12 are defined by two of the longitudinal partitions 28, and a plurality of webs 36b extending in the transverse direction T between the partitions 28.

The wells 16a, 16b may have a closed bottom surface 38a, 38b. Alternatively, the bottom surface of the wells may be open and may include an aperture therethrough.

At least a portion of the inner sidewalls 34a, 34b of the wells 16a, 16b may be discontinuous such that an opening 40 is provided between adjacent wells 16a, 16b in the longitudinal direction. The opening 40 may be defined by the webs 36a, 36b, and may be U-shaped.

The perimeter portion 22 of the upper surface 12 is spatially located above the upper surface of the partitions 28 and the upper surface 42 of the partitions is spatially located above the bottom of the openings 40 defined by the webs 36a, 36b.

The wells 34a, 34b are arranged in rows R1, R2, R3 extending in the longitudinal direction L of the tray 10. The position in the longitudinal direction L of the ends of a first row R1 may be offset from the position in a longitudinal direction L of the ends of a second row R2 adjacent in the transverse direction T to one side of the first row R1 and/or offset from the position in a longitudinal direction L of the ends of a third row R3 adjacent in the transverse direction T to the other side of the first row R1. By offsetting, the rows R1, R2, R3 in this manner, the wells 34b in the first row R1 can be nested between wells 34b in the second row R2 and third row R3.

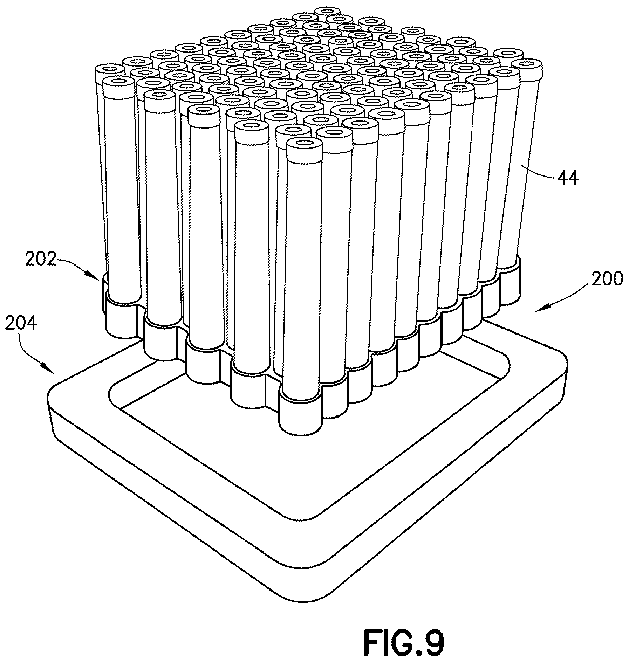

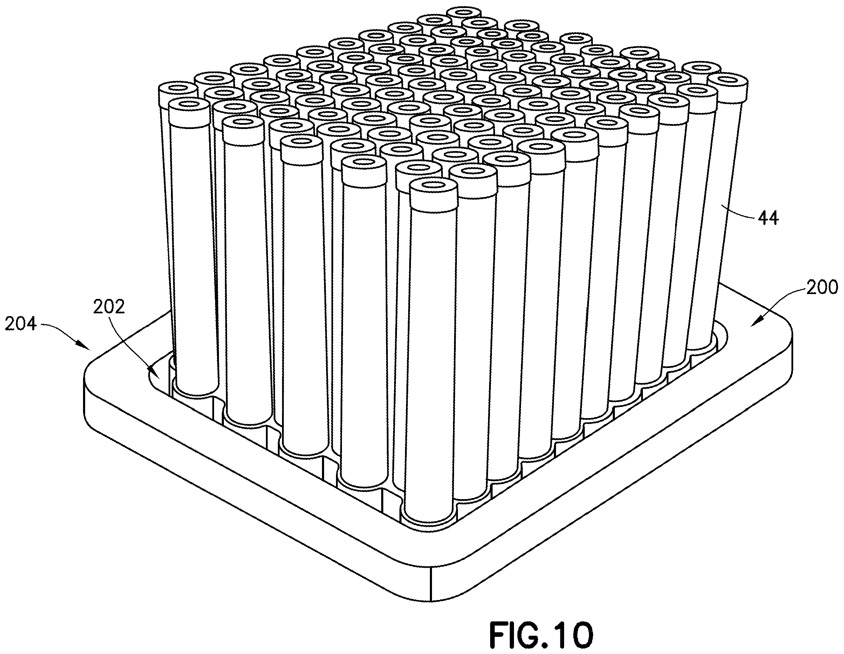

The inner sidewalls 34a, 34b of the wells 16a, 16b may have a shape substantially corresponding the shape of the biological fluid collection container 44 that is to be received by the tray 10. For example, for a collection container 44 having a cylindrical shape with a circular cross-section, as shown in FIGS. 9 and 10, the inner sidewalls 34a, 34b of the wells 16a, 16b may have a substantially cylindrical shape having a circular cross-section. Alternatively, the inner sidewalls of the wells may have a shape that is different from the shape of the collection container, for example, a square, a rectangle, a triangle, an oval, or a polygon, as long as the wells are sized to receive and hold the desired collection container. It is noted herein that the overall size of the tray as described herein may have substantially the same exterior size as a conventional tray formed of expended polystyrene.

The closed bottom surface 38a, 38b may have a shape substantially corresponding the shape of the bottom of the biological fluid collection container 44 that is to be received by the tray 10. For example, for a collection container 44 having semispherical bottom, as shown in FIGS. 9 and 10, the closed bottom surface 38a, 38b of the wells 16a, 16b may have a substantially semispherical bottom surface 38a, 38b. Alternatively, the closed bottom surface of the wells may have a shape that is different from the shape of the collection container.

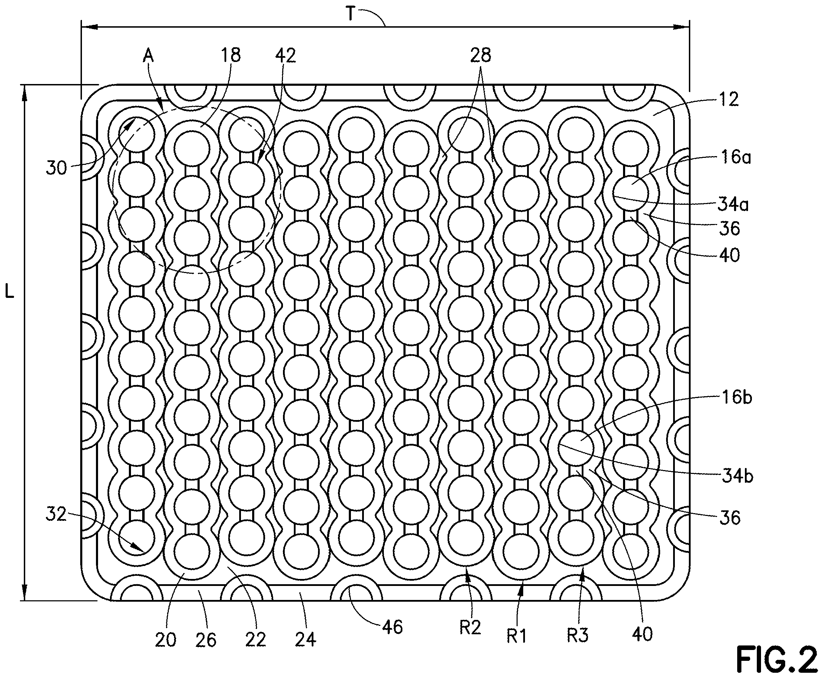

When the tray 10 is configured to hold cylindrical collection containers 44, the inner sidewall 18 has an irregular scalloped shape, and the sides of the partitions 28 have scalloped shapes as shown in FIGS. 1 and 2. The scallops on one side of the partitions 28 are offset from the scallops on the opposite side of the partitions giving the upper surface of the partitions 28 a curved undulating shape as shown in FIG. 2.

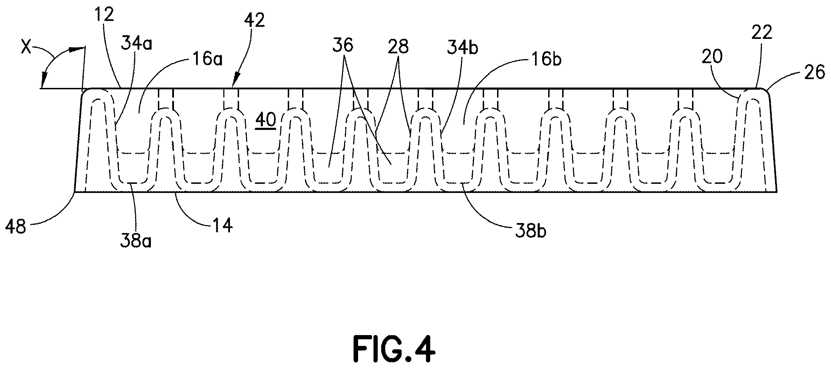

The outer sidewall 24 may be substantially perpendicular to the upper surface 12 or may be angled as shown in FIG. 4. The angle X between an inner surface of the outer sidewall 24 and a bottom surface of the upper surface 12 is greater than 90°, for example, 92°-100° or 93°-96°.

The outer sidewall 24 may include a plurality of ribs 46. The ribs 46 may be provided on one or more sides of the outer sidewall 24 and may be equally spaced along a side or sides of the outer sidewall 24. The ribs 46 may be embossed in the outer sidewall 18 such that the ribs 46 are recesses or concave on the outer surface of the outer sidewall 18 and protrusions or convex on the inner surface of the outer sidewall 18 or the ribs 46 are protrusions or convex on the outer surface of the outer sidewall 18 and recesses or concave on the inner surface of the outer sidewall 18. In this manner, the ribs 46 may provide structural stability to the tray 10 and allow for alignment for nesting of the trays 10 prior to and after use.

The bottom end 48 of the outer sidewall 24 may extend beyond the bottom ends 38a, 38a of the wells 16a, 16b or may be flush with the bottom ends of the wells 16a, 16b as shown in FIG. 4.

The tray 10 is made from plant-based pulp or recycled plant-based pulp molded into a three-dimensional (3D) shape as discussed above.

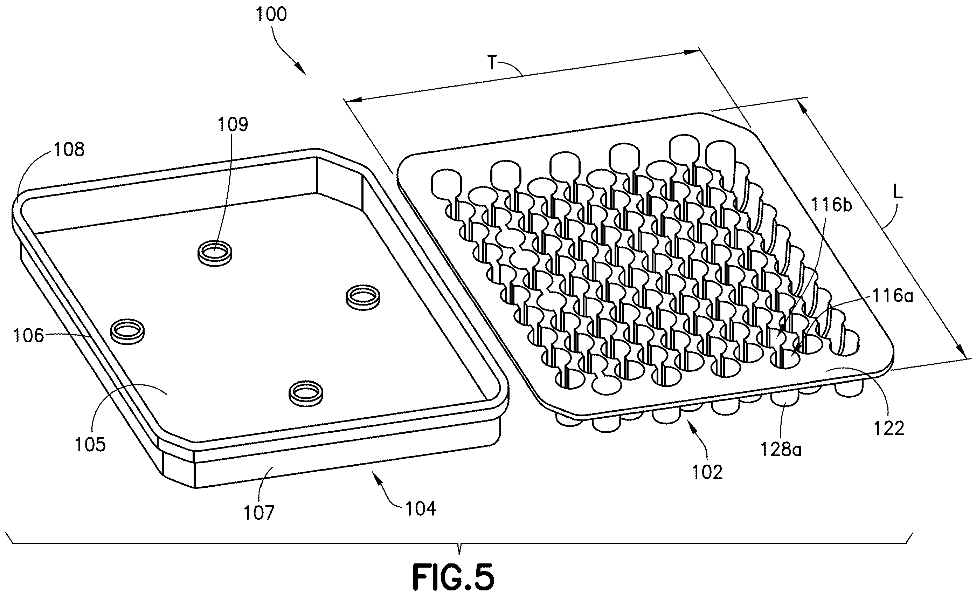

In another embodiment, shown in FIGS. 5 and 6, the tray 100 may be provided in two portions 102, 104. The body 102 of the tray 100 may have the same configuration as the previously described tray 10 except the outer sidewall, which is absent. The base 104 of the tray 100 has a closed bottom end 105, an open top end 106, and a sidewall 107 extending therebetween. The closed bottom end 105 and the sidewall 107 define a compartment 109 adapted to receive the body 102. The upper perimeter of the sidewall 107 has a shape corresponding to the shape of the outer perimeter of the body 102, such that at least a portion of the upper surface 112 of the body 102 acts as a flange allowing the body 102 to be supported by the sidewall 107 of the base 104. A flange 108 may also extend laterally outward from the upper perimeter of the sidewall 107 of the base 104, and at least a portion of the upper surface 112 of the body 102 may be supported by the flange 108. The bottom end 105 of the base 104 may include at least one recess 111 that receives the bottom end 128a, 128b of one of the wells 116a, 116b of the body 102. Both the body 102 and the base 104 of the tray 100 are made from plant-based pulp or recycled plant-based pulp molded into a three-dimensional (3D) shape as discussed above.

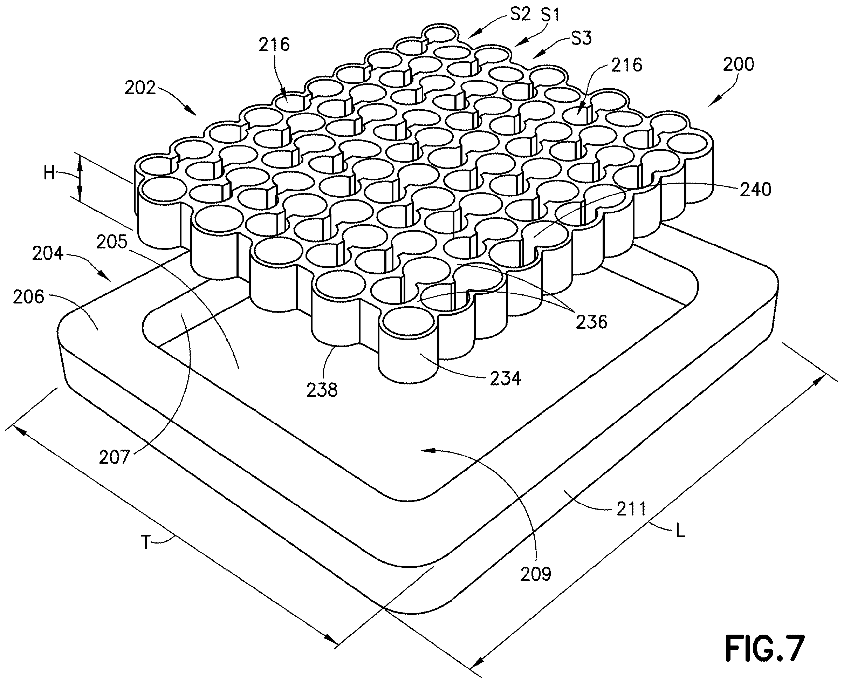

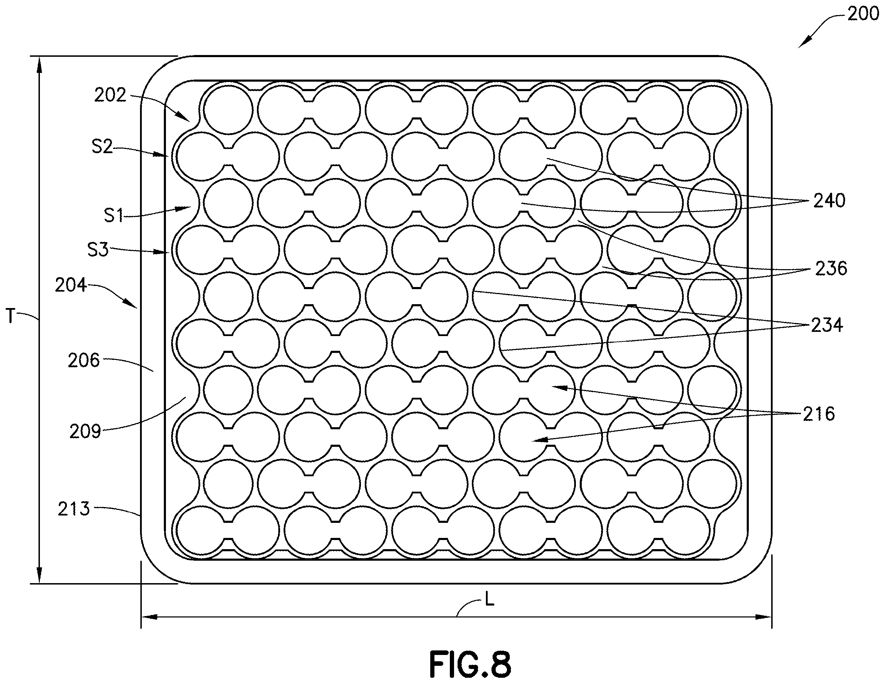

In a further embodiment, the tray 200 may be provided in two parts, 202, 204. As shown in FIGS. 7-10, the container body 202 of the tray 200 comprises a plurality of interconnected wells 216 and has a substantially rectangular or square overall shape.

The wells 216 are arranged in rows S extending in the longitudinal direction L of the tray 10. The position in the longitudinal direction L of the ends of a first row S1 may be offset from the position in a longitudinal direction L of the of the ends of a second row S2 adjacent in the transverse direction T to one side of the first row S1 and/or offset from the position in a longitudinal direction L of the ends of a third row S3 adjacent in the transverse direction T to the other side of the first row S1. By offsetting the rows in this manner, the wells 216 in the first row S1 can be nested between wells 216 in the second row S2 and/or third row S3.

Webs 236 connect the sidewalls 234 of each well to the sidewalls 234 of all adjacent wells 216 in the same row and in the adjacent rows in the transverse direction T1. The webs 236 may extend along the entire height H of the sidewalls 234 of the wells 216, and may include an opening 240 that extends partially or fully along the height H of the sidewalls 234. In the example shown in FIGS. 7-10, every other web 236 in a row S1, S2, S3 contains an opening 240 extending along the entire height H of the sidewalls 234 and no more than one web 236 connected to any well 216 has an opening 240.

The wells 216 may have a closed bottom surface. Alternatively, the bottom surface 238 of the wells 216 may be open.

The wells 216 may have a shape substantially corresponding the shape of the biological fluid collection container 44 that is to be received by the tray 200. For example, for a collection container 44 having a cylindrical shape with a circular cross-section, as shown in FIGS. 9 and 10, the inner portion of the sidewalls 234 of the wells 216 may have a substantially cylindrical shape having a circular cross-section. Alternatively, the inner sidewalls of the wells may have a shape that is different from the shape of the collection container, for example, a square, a rectangle, a triangle, an oval, or a polygon, as long as the wells are sized to receive and hold the desired collection container.

If the bottom surface is closed, the closed bottom surface 238 may have a shape substantially corresponding the shape of the bottom of the biological fluid collection container 44 that is to be received by the tray 200. For example, for a collection container 44 having semispherical bottom, as shown in FIGS. 9 and 10, the closed bottom surface of the wells 216 may have a substantially semispherical bottom surface. Alternatively, the closed bottom surface of the wells may have a shape that is different from the shape of the collection container, and/or may include an aperture therethrough.

The base 204 of the tray 200 has a closed bottom surface 205, a top surface 206, and a sidewall 207 extending therebetween. The closed bottom surface 205 and the sidewall 207 define a compartment 209 adapted to receive the container body 202 of the tray 200. For example, the container body 202 of the tray 200 may have an overall rectangular shape and the compartment 209 may be substantially rectangular. The compartment 209 may have a depth that is equal to, less than, or greater than the height H of the sidewalls 234 of the wells 216. With the container body 202 of the tray 200 received within the compartment 209, the upper surface 210 of the container body 202 of the tray 200 may extend above the top surface 206 of the base 204 of the tray 200, may be below the top surface 206 of the base 204 of the tray 200, or may be flush with the top surface 206 of the base 204 of the tray 200 as shown in FIG. 10.

An outer sidewall 211 may extend from an outer perimeter 213 of the top surface 206 of the base 204. A space is provided between the outer surface of the sidewall 207 and the inner surface of the outer sidewall 211, such that the outer sidewall 211, the top surface 206, and the sidewall 207 have a U-shaped cross-section. Alternatively, the outer portion of the base 204 may have a rectangular cross-section with the sidewall 207, the top surface 206, the outer sidewall 211, and a bottom surface defining the rectangle. The cross-section may be solid or hollow.

The container body 202 of the tray 200 may be molded plastic, for example, thermoplastic materials, such as polyethylene (PE), polyvinylchloride (PVC), polyethylenetheraphthalate (PET), polystyrene (PS), and polypropylene (PP) that has been injection molded. The base 204 of the tray 200 is made from plant-based pulp or recycled plant-based pulp molded into a three-dimensional (3D) shape as discussed above.

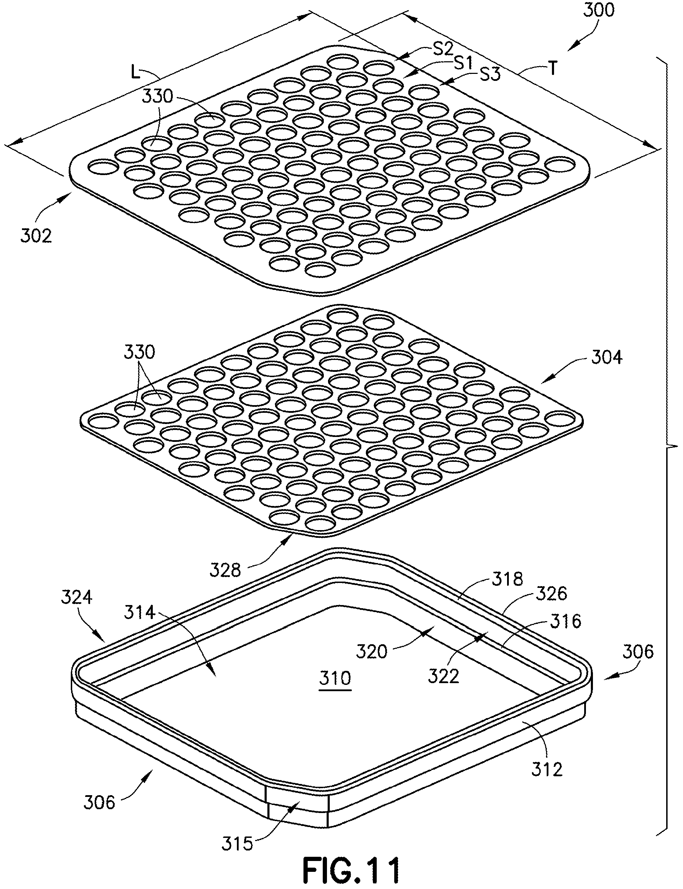

In a further configuration, the tray 300 may be provided in three parts, including two tray inserts 302, 304 and a base 306. As shown in FIGS. 11-12, the tray inserts 302, 304 are arranged in a stacked configuration within base 306, so as to form a plurality of wells 308 within which a plurality of biological fluid collection containers (e.g., the biological fluid collection containers 44 shown in FIGS. 9 and 10) may be received for storage in the tray 300, as explained in detail below.

The base 306 of the tray 300 has a closed bottom surface 310 and a plurality of sidewalls 312 that define a compartment 314 adapted to receive the tray inserts 302, 304. In some configurations, the base 306 may have a substantially rectangular or square overall shape. Further, in some embodiments, the base 306 is configured to have a pair of opposing corners 315 (defined by sidewalls) having a chamfered construction.

The sidewalls 312 of base 306 may have a stepped construction, such that a pair of ledges 316, 318 is formed on the base 306 that each extend about a perimeter thereof. A first ledge 316 is positioned between a lower portion 320 of sidewalls 312 and an upper region 322 of sidewalls 312, approximately halfway between the bottom surface 310 and a top surface 324 of the base 306. The first ledge 316 provided on sidewalls 312 serves to position the upper region 322 of the sidewalls 312 further outward from the lower region 320 of the sidewalls 312. A second ledge 318 is positioned adjacent the top surface 324 of base 306 (i.e., adjacent an upper edge of upper region 322), with a small outer lip 326 of the sidewalls 312 set outward from the second ledge 318 and extending up beyond the second ledge 318. As shown best in FIG. 11, the second ledge 318 is spaced outwardly from first ledge 316, thereby enabling receiving of the tray inserts 302, 304 in a stacked configuration when positioned in base 306.

Each of the tray inserts 302, 304 has a planar construction and a substantially rectangular or square overall shape that matches that of base 306. Thus, in some configurations, tray inserts 302, 304 are configured to have a pair of opposing corners 328 having a chamfered construction, so as to match and align with chamfered corners 315 of base 306. The tray inserts 302, 304 are of a similar construction, except that bottom tray insert 304 has a slightly smaller size than that of top tray insert 302. The bottom tray insert 304 is sized so as to be received within the compartment 314 of base 306, with the bottom tray insert 304 sized and positioned so as to rest on first ledge 316 of sidewalls 312. The top tray insert 302 is sized so as to be retained in position adjacent the top surface 324 of base 306, with the top tray insert 302 sized and positioned so as to rest on second ledge 318 of sidewalls 312, such that top tray insert 302 may be flush with the top surface 324 of the base 306, as shown in FIG. 11, or may be recessed downward by a small amount from the top surface 324 of the base 306. With the tray inserts 302, 304 received in base 306, the top tray insert 302 is thus spaced vertically apart from the bottom tray insert 304.

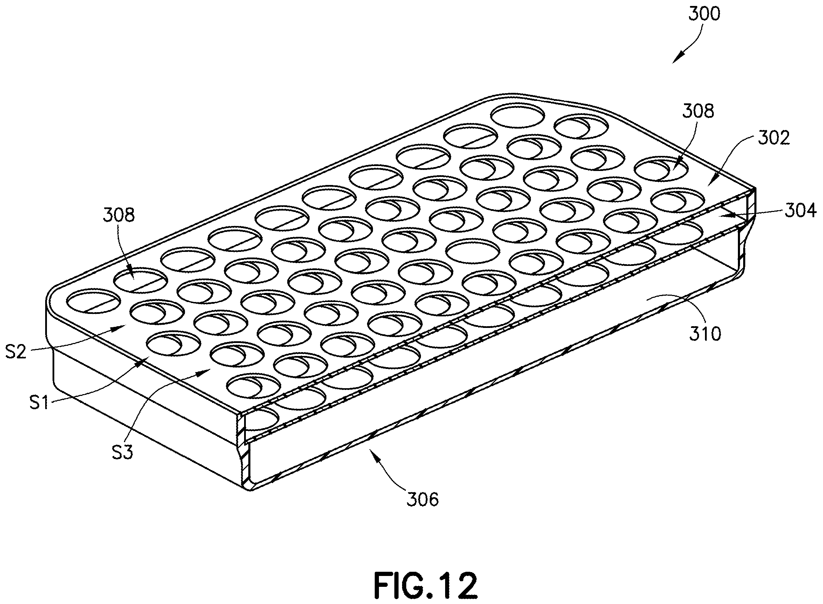

As shown in FIGS. 11 and 12, each of the tray inserts 302, 304 includes a plurality of openings 330 therein. The openings 330 may have a shape substantially corresponding to the shape of the biological fluid collection container 44 that is to be received by the tray 300. For example, for a collection container 44 having a cylindrical shape with a circular cross-section, as shown in FIGS. 9 and 10, the openings 330 in tray inserts 302, 304 may have a circular cross-section. Alternatively, the openings 330 in tray inserts 302, 304 may have a shape that is different from the shape of the collection container 44, for example, a square, a rectangle, a triangle, an oval, or a polygon, as long as the openings 330 are sized to receive and hold the desired collection container.

With the tray inserts 302, 304 arranged in a stacked configuration when positioned in base 306, the top tray insert 302 is spaced vertically apart from the bottom tray insert 304 and the openings 330 in the top tray insert 302 are aligned with the openings 330 in the bottom tray insert 304, such that a plurality of wells 308 are defined in the tray 300 that may retain the biological fluid collection containers 44 therein. The wells 308 (and openings 330 in tray inserts 302, 304 that, when aligned, define the wells 308) are arranged in rows S extending in the longitudinal direction L of the tray 300. The position in the longitudinal direction L of the ends of a first row S1 may be offset from the position in a longitudinal direction L of the of the ends of a second row S2 adjacent in the transverse direction T to one side of the first row S1 and/or offset from the position in a longitudinal direction L of the ends of a third row S3 adjacent in the transverse direction T to the other side of the first row S1. By offsetting the rows in this manner, the wells 308 in the first row S1 can be nested between wells 308 in the second row S2 and/or third row S3.

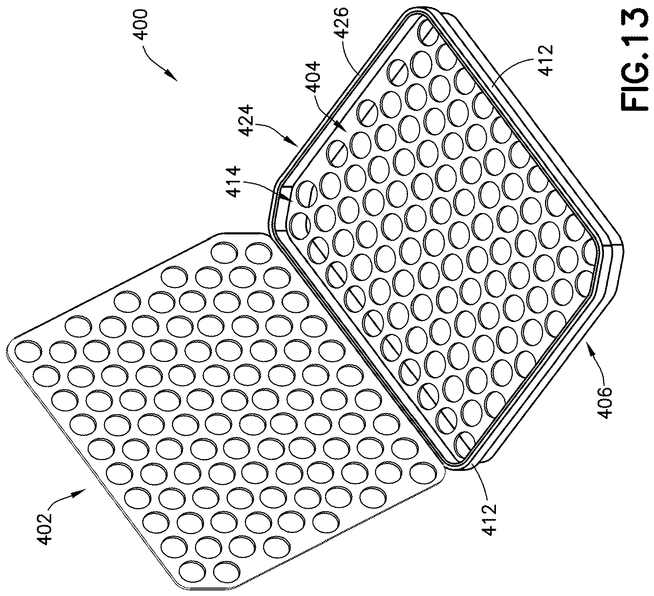

In some configurations, and as shown in FIG. 13, a tray 400 may be provided where one or both of the tray inserts 402, 404 (as described above in FIGS. 11 and 12) are formed integrally with the base 406 rather than as separate components. That is, one or both of top tray insert 402 and bottom tray insert 404 may be provided as a hinged insert that is coupled to the base 406 along one edge or side surface thereof. The integrating of one or both of the tray inserts 402, 404 with the base 406 may reduce the number of separate and distinct components in the tray 400 and reduce the number of manufacturing steps required to form the tray 400, so as by reducing the number of thermoforming or injection molding steps required in the manufacturing process.

In some configurations, the bottom tray insert 404 may be formed integrally with base 406 such that one side surface thereof is attached to a sidewall 412 of the base 406. The attachment/forming of bottom tray insert 404 to the sidewall 412 provides for a hinge-type feature about which the bottom tray insert 404 may be rotated. In some configurations, the top tray insert 402 may be formed integrally with base 406 such that one side surface thereof is attached to a sidewall 412 of the base 406 adjacent the lip 426 provided at the top surface 424 of the base 406. The attachment/forming of top tray insert 402 to the sidewall 412 in the region of lip 426 provides for a hinge-type feature about which the top tray insert 402 may be rotated. With the top tray insert 402 and/or bottom tray insert 404 rotatable away from the base 406, access may be provided to the compartment 414 defined by the base 406.

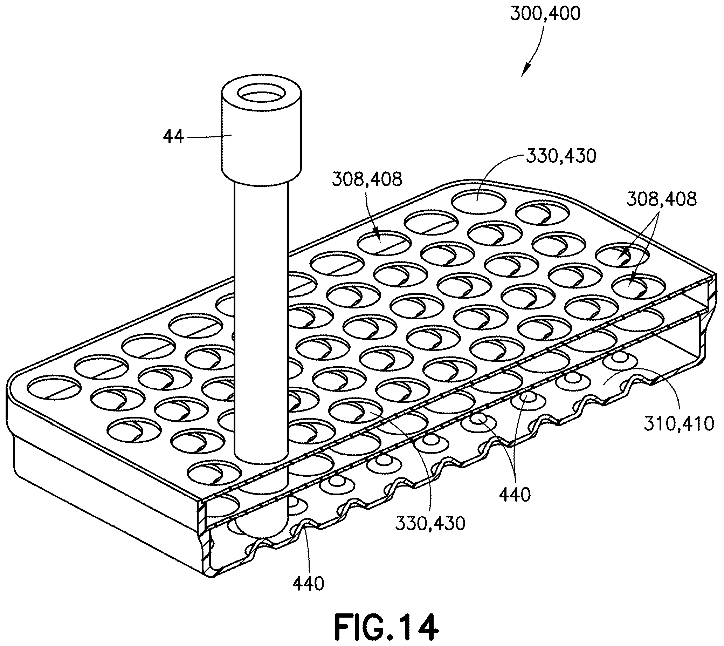

In either of the tray 300 of FIGS. 11-12 or the tray 400 of FIG. 13, retaining features may be added to the closed bottom surface 310, 410 to provide an additional mechanism for supporting and retaining fluid collection containers 44 in place. As shown in FIG. 14, in some configurations, the retaining features are configured as nubs or dimples 440 that are formed (e.g., molded) integrally with the bottom surface 310, 410 and that extend upwardly from the bottom surface 310, 410. The dimples 440 are aligned with the wells 308, 408 formed by top tray insert 302, 402 and bottom tray insert 402, 404 (i.e., by openings 330, 430 thereof), such that the dimples 440 are positioned directly under the fluid collection containers 44, with a dimple 440 provided for each well 308, 408 in tray 300, 400. In some configurations, the dimples 440 may have a domed type configuration, where the dome depresses and deforms inwardly if a downward pressure is applied thereto. The dimples 440 thus provide support and cushioning to the (semispherical) bottom of the biological fluid collection containers 44, to provide additional packaging protection to the containers 44 when packed and stored within the tray 300, 400.

According to the configurations of FIGS. 11-14, each of the tray inserts 302, 304, 402, 404 and the base 306, 406 may be made from plant-based pulp or recycled plant-based pulp molded into a three-dimensional (3D) shape, as discussed above. The tray inserts 302, 304, 402, 404 and the base 306, 406 may be made by thermoforming or injection molding, and additives, such as hydrophobic additives or fillers, may be included to provide the finished tray with moisture resistance and/or strength. In other configurations only one of the tray inserts 302, 304, 402, 404 or the base 306, 406 may be made from plant-based pulp or recycled plant-based pulp.

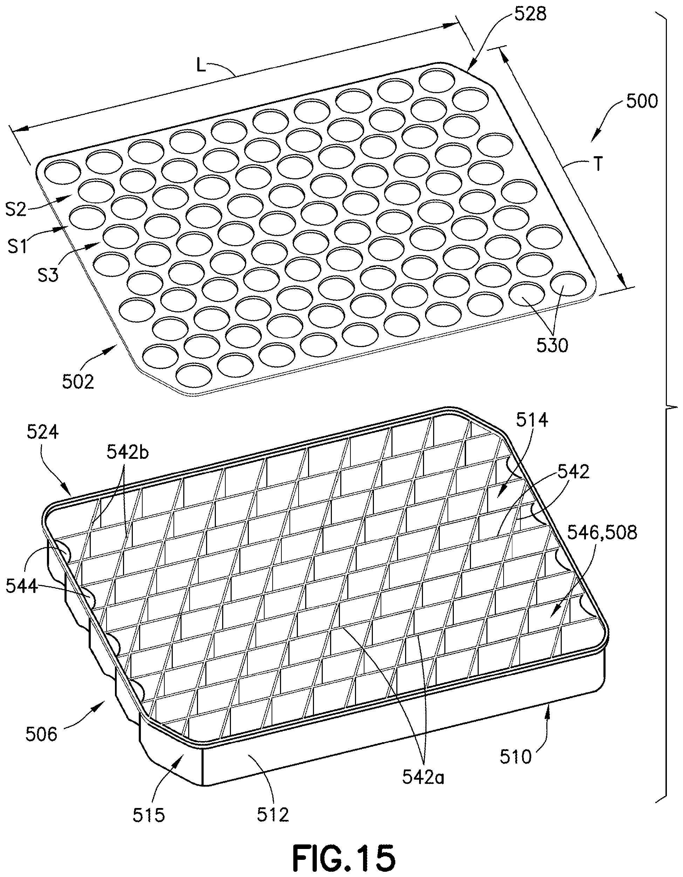

Referring now to FIG. 15, a tray 500 is provided that includes a tray insert 502 and a base 506. The tray insert 502 may be configured as previously described regarding the tray inserts 302, 304 of tray 300, with the tray insert 502 having a planar construction and a substantially rectangular or square overall shape and that include a pair of opposing corners 528 having a chamfered construction. The tray insert 502 includes a plurality of openings 530 therein that have a shape substantially corresponding to the shape of the biological fluid collection container 44, with the openings 530 in tray insert 502 having a circular cross-section in one configuration. The openings 530 are arranged in rows S extending in the longitudinal direction L of the tray 500. The position in the longitudinal direction L of the ends of a first row S1 may be offset from the position in a longitudinal direction L of the of the ends of a second row S2 adjacent in the transverse direction T to one side of the first row S1 and/or offset from the position in a longitudinal direction L of the ends of a third row S3 adjacent in the transverse direction T to the other side of the first row S1. By offsetting the rows in this manner, the openings 530 in the first row S1 can be nested between openings 530 in the second row S2 and/or third row S3.

As shown in FIG. 15, the base of the tray 500 has a closed bottom surface 510 and a plurality of sidewalls 512 that define a compartment 514, as well as a plurality of interior walls 542 arranged within the compartment 514 in a crisscrossing arrangement. The base 506 may have a substantially rectangular or square overall shape that matches that of tray insert 502 and that, in the illustrated configuration, has opposing corners 515 having a chamfered construction to match that of tray insert 502. An opposing pair of sidewalls 512 of base 506 (e.g., opposing longitudinal sides) may have bump-outs 544 formed thereon that extend inwardly from a main surface of the sidewall 512, with the bump-outs 544 providing support to the tray insert 502, which may rest thereon when provided on the base 506.

The plurality of interior walls 542 includes a first set of walls 542a and a second set of walls 542b arranged within the compartment in a crisscrossing arrangement, with each of the walls 542 from the first and second sets of walls 542a, 542b extending generally from the bottom surface 510 of base 506 to the top surface 524 of base 506. The first set of walls 542a may be arranged in parallel with the rows S of openings 530 formed in the tray insert 502 that extend in the longitudinal direction L of the tray 10, with each wall 542 in the first set of walls 542a positioned between adjacent rows of openings 530 so as to not obstruct any of the openings 530. The second set of walls 542b may be arranged at a non-perpendicular angle to the first set of walls 542a, such that each wall 542 in the second set of walls 542b crosses a plurality of the walls 542 of the first set of walls 542a at an angle. Each wall 542 in the second set of walls 542b may be angled relative to the first set of walls 542a based on the offset in the longitudinal direction L between adjacent pairs of rows S of openings 530 in tray insert 502 - with the second set of walls 542b being angled such that they match an angle between offset openings 530 in adjacent rows. The walls 542 of the second set of walls 542b are thus oriented so as to not obstruct any of the openings 530 in the tray insert 502.

Resulting from the crisscrossing arrangement of the first set of walls 542a and the second set of walls 542b, a plurality of receptacles 546 are provided within the compartment 514 of base 506, with each receptacle 546 having a parallelogram shape defined by a pair of walls 542 from the first set of walls 542a and a pair of walls 542 from the second set of walls 542b. Each receptacle 546 of the base 506 is aligned with an associated opening 530 formed in the tray insert 502 such that, with the tray insert 502 positioned on the base 506, an opening 530 and its associated receptacle 546 together form a well 508 within which a biological fluid collection container (e.g., the biological fluid collection container 44 shown in FIGS. 9 and 10) may be received for storage in the tray 500. As described above regarding the openings 530 in tray insert 502, the wells 508 provided in tray 500 (formed by associated openings 530 and receptacles 546) are offset in adjacent rows, such that the wells 508 in the first row S1 can be nested between wells 508 in the second row S2 and/or third row S3.

According to configurations, each of the tray insert 502 and the base 506 is made from plant-based pulp or recycled plant-based pulp molded into a three-dimensional (3D) shape, as discussed above. The tray insert 502 and the base 506 may be made by thermoforming or injection molding, and additives, such as hydrophobic additives or fillers, may be included to provide the finished tray with moisture resistance and/or strength.

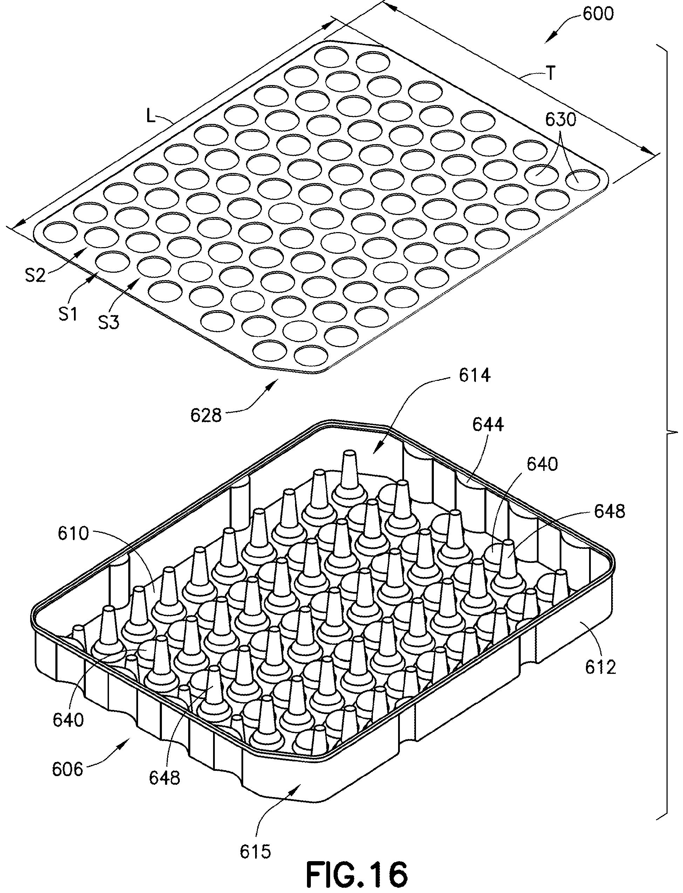

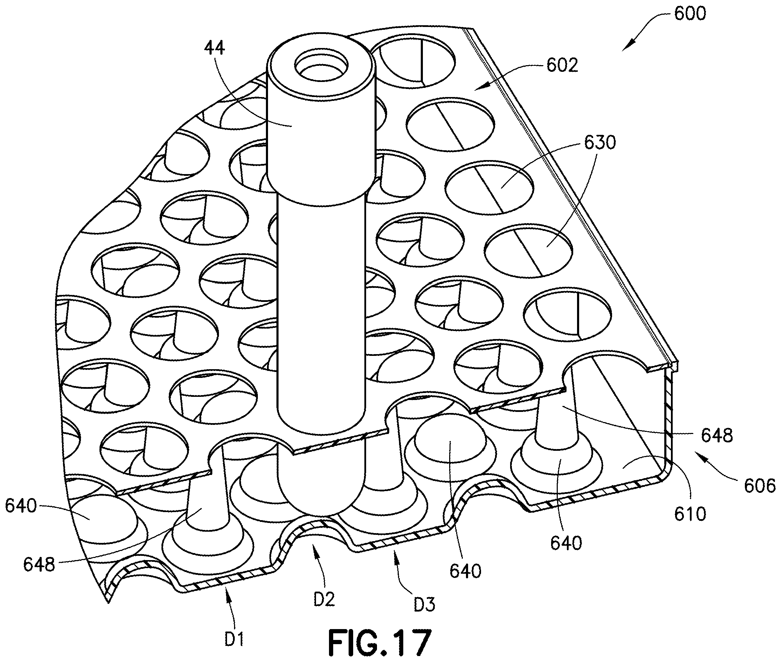

Referring now to FIGS. 16 and 17, a tray 600 is provided that includes a tray insert 602 and a base 606. The tray insert 602 may be configured as previously described regarding the tray insert 502 of tray 500, with the tray insert 602 having a planar construction and a substantially rectangular or square overall shape and that include a pair of opposing corners 628 having a chamfered construction. The tray insert 602 includes a plurality of openings 630 therein arranged in rows S extending in the longitudinal direction L of the tray 600. The position in the longitudinal direction L of the ends of a first row S1 may be offset from the position in a longitudinal direction L of the of the ends of a second row S2 adjacent in the transverse direction T to one side of the first row S1 and/or offset from the position in a longitudinal direction L of the ends of a third row S3 adjacent in the transverse direction T to the other side of the first row S1. By offsetting the rows in this manner, the openings 630 in the first row S1 can be nested between openings 630 in the second row S2 and/or third row S3.

As shown in FIG. 16, the base 606 of the tray 600 has a closed bottom surface 610 and a plurality of sidewalls 612 that define a compartment 614. The base 606 may have a substantially rectangular or square overall shape that matches that of tray insert 602 and that, in the illustrated configuration, has opposing corners 615 having a chamfered construction to match that of tray insert 602. At least some of the sidewalls 612 may have bump-outs 644 formed thereon that extend inwardly from a main surface of the sidewall, with the bump-outs 644 providing support to the tray insert 602, which may rest thereon when provided on the base 606.

The bottom surface 610 of base 606 includes thereon retaining features to provide a mechanism for supporting and retaining fluid collection containers 44 in place. As best shown in FIG. 17, the retaining features are configured as nubs or dimples 640 that are formed (e.g., molded) integrally with the bottom surface 610 and that extend upwardly from the bottom surface 610. In some configurations, the dimples 640 may have a domed type configuration, where the dome depresses and deforms inwardly if a downward pressure is applied thereto. The number of dimples 640 provided on bottom surface 610 may correspond to the number of openings 630 formed in tray insert 602, but the dimples 640 are offset from the openings 630, such that each dimple 640 is positioned between multiple openings 630 of the tray insert 602. With the dimples 640 being offset from openings 630, a fluid collection container 44 inserted within a respective opening 630 extends down and makes partial contact with multiple dimples 640 of base 606. In one configuration, a fluid collection container 44 inserted within a respective opening 630 makes contact with three surrounding dimples 640, such that three points of contact are made with the fluid collection container 44 that provide support and cushioning to the (semispherical) bottom thereof, to provide additional packaging protection to the container 44 when packed and stored within the tray 600. Each respective opening 630 and the respective collection of three dimples 640 that collectively receive a fluid collection container 44 therein may be considered a "well" in tray 600.

As shown in FIGS. 16 and 17, a portion of the dimples 640 include a support post 648 that extends upwardly therefrom to make contact with a bottom surface 610 of tray insert 602 when the tray insert 602 is positioned on base 606. Each support post 648 may be formed integrally with the dimple 640 from which it extends. Each support post 648 may be configured as a generally cylindrical structure, although the support post 648 may taper from a thicker bottom portion (adjacent dimple 640) to a more slender upper portion (adjacent tray insert 602). The support posts 648 provide points of contact between the base 606 and the tray insert 602 to provide additional support and structural integrity to the tray insert 602. The support posts 648 are shaped and positioned on dimples 640 such that, when a respective fluid collection container 44 is inserted within an opening 630 in tray insert 602 and extends down to make three points of contact with surrounding dimples 640, the container 44 does not come into contact with any of support posts 648.

As indicated above, in some configurations, support posts 648 are provided on only a portion of the dimples 640 of base 606. In one configuration, and as best shown in FIG. 17, support posts 648 are provided on dimples 640 only on every other longitudinal row D of dimples 640, such that, for example, a first row D1 and a third row D3 of dimples 640 includes support posts 648 thereon, while a second row D2 of dimples 640 between the first and third rows D1, D3 does not include support posts 648 thereon. In other configurations, alternative arrangements of support posts 648 may be provided on base 606, such as support posts 648 being provided on every other dimple in each row D1, D2, D3 of dimples 640. In still other alternate configurations, it is recognized that each of dimples 640 could include a support post 648 thereon.

In all embodiments, the wells may be adapted to receive blood collection tubes having diameters of 8-16 mm, for example, Vacutainer^{®} blood collection tubes, Vacutainer^{®} CPT^{™} blood collection tubes, Vacutainer^{®} PPT^{™} blood collection tubes, Microtainer^{®} blood collection tubes, and Microtainer^{®} MAP blood collection tubes.

Unlike prior art expanded polystyrene (EPS) trays and other plastic trays, the inventive pulp trays are recyclable, biodegradable, compostable, and recoverable as energy. The inventive trays can be recycled in substantially any paper recycling stream and can be recycled with paper, corrugated boxes, office waste, etc. The trays conform to EN 13430:2004 entitled "Packaging - Requirements for Packaging Recoverable by Material Recycling".

In addition, the inventive trays have high strength and rigidity and are lightweight, compact, easy to inspect for damage and defects, inexpensive to manufacture with good dimensional stability, and easily transported.

Whereas particular aspects of this invention have been described above for purposes of illustration, it will be evident to those skilled in the art that numerous variations of the details of the present invention may be made without departing from the invention, which is defined in the appended claims.

## Claims

1. A tray (100) for storing biological fluid collection containers (44) comprising:
a body (102) comprising:
an upper surface (112) comprising a perimeter portion (22) having an outer perimeter (26) and an inner perimeter (20); and
a recess extending downwardly from and surrounded by the inner perimeter (20) of the perimeter portion (22),
wherein the recess is divided into a plurality of wells (116a, 116b), each well adapted to receive at least a portion of a biological fluid collection container (44), and
a base (104) comprising:
a closed bottom end (105);
an open top end (106); and
a sidewall (107) extending therebetween,
wherein the closed bottom end (105) and the sidewall (107) of the base (104) define a compartment (109) that receives the body (102), and
**characterized in that**
the closed bottom end (105) of the base (104) includes at least one recess (111) that receives a bottom end (128a, 128b) of one of the plurality of wells (116a, 116b); and
wherein the body (102) and the base (104) of the tray (100) comprise molded plant-based pulp.

2. The tray (100) according to claim 1, wherein inner sidewalls (34a, 34b) of the plurality of wells (116a, 116b) adjacent to the perimeter portion (22) are defined by a body inner sidewall (18) extending downwardly from the inner perimeter (20) of the perimeter portion (22), a longitudinal partition (28) extending in a longitudinal direction (L) from a first side (30) of the body inner sidewall (18) to an opposing second side (32) of the body inner sidewall (18), and a plurality of webs (36a, 36b) extending in a transverse direction (T) between the body inner sidewall (18) and the partition (28).

3. The tray (100) according to claim 1, wherein a body inner sidewall (18) extends downwardly from the inner perimeter (20) of the perimeter portion (22) and inner sidewalls (34a, 34b) of the plurality of wells (116a, 116b) that are not adjacent to the perimeter portion (22) are defined by two longitudinal partitions (28) extending in a longitudinal direction (L) from a first side (30) of the body inner sidewall (18) to an opposing second side (32) of the body inner sidewall (18), and a plurality of webs (36a, 36b) extending in a transverse direction (T) between the partitions (28).

4. The tray (100) according to claim 1, wherein the plurality of wells (116a, 116b) have a closed bottom surface (38a, 38b) and wherein preferably at least a portion of inner sidewalls (34a, 34b) of the plurality of wells (116a, 116b) are discontinuous such that an opening (40) is provided between adjacent wells (116a, 116b).

5. The tray (100) according to claim 1, wherein a plurality of wells (116a, 116b) are defined by a body inner sidewall (18) extending downwardly from the outer perimeter (26) of the perimeter portion (22), a longitudinal partition (28) extending in a longitudinal direction (L) from a first side (30) of the body inner sidewall (18) to an opposing second side (32) of the body inner sidewall (18), and a plurality of webs (36a, 36b) extending in a transverse direction (T) between the body inner sidewall (18) and the partition (28) or by two longitudinal partitions (28) extending in a longitudinal direction (L) from a first side (30) of a body inner sidewall (18) extending downwardly from the inner perimeter (20) of the perimeter portion (22) to an opposing second side (32) of the body inner sidewall (18), and a plurality of webs (36a, 36b) extending in a transverse direction (T) between the partitions (28), and the opening (40) is defined by the webs (36a, 36b).

6. The tray (100) according to claim 1, wherein the plurality of wells (116a, 116b) are arranged in rows (R1, R2, R3) extending in the longitudinal direction (L) of the tray (100).

7. The tray (100) according to claim 6, wherein a position in the longitudinal direction (L) of ends of a first row (R1) are offset from a position in a longitudinal direction (L) of ends of a second row (R2) adjacent in the transverse direction (T) to one side of the first row (R1) and/or offset from a position in the longitudinal direction (L) of ends of a third row (R3) adjacent in the transverse direction (T) to an opposite side of the first row (R1).

8. The tray (100) according to claim 1, wherein inner sidewalls (34a, 34b) of the plurality of wells (116a, 116b) have a shape substantially corresponding a shape of the biological fluid collection container (44) that is to be received by the tray (100) and/or wherein closed bottom surfaces (38a, 38b) of the plurality of wells (116a, 116b) have a shape substantially corresponding a shape of a bottom surface of the biological fluid collection container (44) that is to be received by the tray (100) and/or wherein the plurality of wells (116a, 116b) are adapted to hold cylindrical biological fluid collection containers (44).

9. The tray (100) according to claim 1, further comprising an outer sidewall (24) extending downwardly from the outer perimeter (26) of the perimeter portion (22).

10. The tray (100) according to claim 9, wherein the outer sidewall (24) is substantially perpendicular to the upper surface (112) or wherein the outer sidewall (24) is angled with respect to the upper surface (112) or wherein the outer sidewall (24) includes a plurality of ribs (46).

11. The tray (100) according to claim 10, wherein the ribs (46) are embossed in the outer sidewall (24) such that the ribs (46) are recesses on an outer surface of the outer sidewall (24) and protrusions on an inner surface of the outer sidewall (24) or the ribs (46) are protrusions on an outer surface of the outer sidewall (24) and recesses on an inner surface of the outer sidewall (24).

12. The tray (100) according to claim 1, wherein the base (104) further comprises a flange (108) extending laterally outward from an upper perimeter of the sidewall (107) of the base (104), and at least a portion of the perimeter portion (22) of the body (102) is supported by the flange (108) when the body (102) is received in the compartment (109) of the base (104).

13. The tray (100) according to claim 1, wherein the tray (100) comprises molded recycled plant-based pulp.

14. A method for manufacturing the tray (100) according to claim 1, comprising molding the tray (100) from plant-based pulp or recycled plant-based pulp by thermoforming moistened plant-based pulp or recycled plant-based pulp.

15. A method for manufacturing the tray (100) according to claim 1, comprising molding the tray (100) from plant-based pulp or recycled plant-based pulp by injection molding moistened plant-based pulp or recycled plant-based pulp.

## Patentansprüche

1. Schale (100) zur Aufbewahrung von Entnahmebehältern (44) für biologische Fluide, aufweisend:
einen Körper (102), der aufweist:
eine Oberseite (112), die einen Umfangsbereich (22) mit einem Außenumfang (26) und einem Innenumfang (20) aufweist; und
eine Aussparung, die sich vom Innenumfang (20) des Umfangsbereichs (22) nach unten erstreckt und von diesem umgeben ist,
wobei die Aussparung in eine Vielzahl von Vertiefungen (116a, 116b) unterteilt ist, wobei jede Vertiefung so ausgelegt ist, dass sie zumindest einen Abschnitt eines Entnahmebehälters (44) für biologische Fluide aufnehmen kann, und
eine Basis (104), die aufweist:
ein geschlossenes unteres Ende (105);
ein offenes oberes Ende (106); und
eine sich dazwischen erstreckende Seitenwand (107),
wobei das geschlossene untere Ende (105) und die Seitenwand (107) der Basis (104) ein Fach (109) definieren, das den Körper (102) aufnimmt, und
**dadurch gekennzeichnet, dass**
das geschlossene untere Ende (105) der Basis (104) mindestens eine Aussparung (111) aufweist, die ein unteres Ende (128a, 128b) einer der Vielzahl von Vertiefungen (116a, 116b) aufnimmt; und
wobei der Körper (102) und die Basis (104) der Schale (100) aus geformtem pflanzlichem Zellstoff bestehen.

2. Schale (100) nach Anspruch 1, wobei die inneren Seitenwände (34a, 34b) der Vielzahl von Vertiefungen (116a, 116b), die an den Umfangsbereich (22) angrenzen, definiert werden durch eine innere Körper-Seitenwand (18), die sich vom Innenumfang (20) des Umfangsbereichs (22) nach unten erstreckt, eine Längstrennwand (28), die sich in Längsrichtung (L) von einer ersten Seite (30) der inneren Körper-Seitenwand (18) zu einer entgegengesetzten zweiten Seite (32) der inneren Körper-Seitenwand (18) erstreckt, und eine Vielzahl von Stegen (36a, 36b), die sich in einer Querrichtung (T) zwischen der inneren Körper-Seitenwand (18) und der Trennwand (28) erstrecken.

3. Schale (100) nach Anspruch 1, wobei sich eine innere Körper-Seitenwand (18) vom Innenumfang (20) des Umfangsbereichs (22) nach unten erstreckt und innere Seitenwände (34a, 34b) der Vielzahl von Vertiefungen (116a, 116b), die nicht an den Umfangsbereich (22) angrenzen, durch zwei Längstrennwände (28) definiert werden, die sich in Längsrichtung (L) von einer ersten Seite (30) der inneren Körper-Seitenwand (18) zu einer entgegengesetzten zweiten Seite (32) der inneren Körper-Seitenwand (18) erstrecken, sowie durch eine Vielzahl von Stegen (36a, 36b), die sich in einer Querrichtung (T) zwischen den Trennwänden (28) erstrecken.

4. Schale (100) nach Anspruch 1, wobei die Vielzahl von Vertiefungen (116a, 116b) eine geschlossene Unterseite (38a, 38b) aufweisen und wobei vorzugsweise zumindest ein Teil der inneren Seitenwände (34a, 34b) der Vielzahl von Vertiefungen (116a, 116b) unterbrochen sind, sodass zwischen benachbarten Vertiefungen (116a, 116b) eine Öffnung (40) vorgesehen ist.

5. Schale (100) nach Anspruch 1, wobei eine Vielzahl von Vertiefungen (116a, 116b) definiert werden durch eine innere Körper-Seitenwand (18), die sich vom Außenumfang (26) des Umfangsbereichs (22) nach unten erstreckt, eine Längstrennwand (28), die sich in Längsrichtung (L) von einer ersten Seite (30) der inneren Körper-Seitenwand (18) zu einer entgegengesetzten zweiten Seite (32) der inneren Körper-Seitenwand (18) erstreckt, und eine Vielzahl von Stegen (36a, 36b), die sich in einer Querrichtung (T) zwischen der inneren Körper-Seitenwand (18) und der Trennwand (28) erstrecken oder durch zwei Längstrennwände (28), die sich in einer Längsrichtung (L) von einer ersten Seite (30) einer inneren Körper-Seitenwand (18), die sich vom Innenumfang (20) des Umfangsbereichs (22) nach unten erstreckt, zu einer entgegengesetzten zweiten Seite (32) der inneren Körper-Seitenwand (18) erstreckt, und eine Vielzahl von Stegen (36a, 36b), die sich in einer Querrichtung (T) zwischen den Trennwänden (28) erstrecken, und wobei die Öffnung (40) durch die Stege (36a, 36b) definiert wird.

6. Schale (100) nach Anspruch 1, wobei die Vielzahl von Vertiefungen (116a, 116b) in Reihen (R1, R2, R3) angeordnet sind, die sich in Längsrichtung (L) der Schale (100) erstrecken.

7. Schale (100) nach Anspruch 6, wobei eine Position in Längsrichtung (L) von Enden einer ersten Reihe (R1) gegenüber einer Position in Längsrichtung (L) von Enden einer zweiten Reihe (R2) versetzt ist, die in Querrichtung (T) an eine Seite der ersten Reihe (R1) angrenzen, und/oder gegenüber einer Position in Längsrichtung (L) von Enden einer dritten Reihe (R3) versetzt ist, die in Querrichtung (T) an einer gegenüberliegenden Seite der ersten Reihe (R1) angrenzt.

8. Schale (100) nach Anspruch 1, wobei innere Seitenwände (34a, 34b) der Vielzahl von Vertiefungen (116a, 116b) eine Form aufweisen, die im Wesentlichen einer Form des von der Schale (100) aufzunehmenden Entnahmebehälters (44) für biologische Fluide entspricht, und/oder wobei geschlossene Unterseiten (38a, 38b) der Vielzahl von Vertiefungen (116a, 116b) eine Form aufweisen, die im Wesentlichen einer Form einer Unterseite des von der Schale (100) aufzunehmenden Entnahmebehälters (44) für biologische Fluide entspricht, und/oder wobei die Vielzahl von Vertiefungen (116a, 116b) so ausgelegt sind, dass sie zylindrische Entnahmebehälter (44) für biologische Fluide halten können.

9. Schale (100) nach Anspruch 1, die ferner eine äußere Seitenwand (24) aufweist, die sich vom Außenumfang (26) des Umfangsbereichs (22) nach unten erstreckt.

10. Schale (100) nach Anspruch 9, wobei die äußere Seitenwand (24) im Wesentlichen senkrecht zur Oberseite (112) steht oder wobei die äußere Seitenwand (24) in Bezug auf die Oberseite (112) abgewinkelt ist oder wobei die äußere Seitenwand (24) eine Vielzahl von Rippen (46) aufweist.

11. Schale (100) nach Anspruch 10, wobei die Rippen (46) in die äußere Seitenwand (24) eingeprägt sind, sodass die Rippen (46) an einer Außenfläche der äußeren Seitenwand (24) Aussparungen sind und an einer Innenfläche der äußeren Seitenwand (24) Vorsprünge sind oder die Rippen (46) an einer Außenfläche der äußeren Seitenwand (24) Vorsprünge sind und an einer Innenfläche der äußeren Seitenwand (24) Aussparungen sind.

12. Schale (100) nach Anspruch 1, wobei die Basis (104) ferner einen Flansch (108) aufweist, der sich seitlich nach außen von einem oberen Umfang der Seitenwand (107) der Basis (104) erstreckt, und wobei zumindest ein Teil des Umfangsbereichs (22) des Körpers (102) von dem Flansch (108) abgestützt wird, wenn der Körper (102) in dem Fach (109) der Basis (104) aufgenommen ist.

13. Schale (100) nach Anspruch 1, wobei die Schale (100) aus geformtem recyceltem pflanzlichem Zellstoff besteht.

14. Verfahren zur Herstellung der Schale (100) nach Anspruch 1, welches das Formen der Schale (100) aus pflanzlichem Zellstoff oder recyceltem pflanzlichem Zellstoff durch Thermoformen von angefeuchtetem pflanzlichem Zellstoff oder recyceltem pflanzlichem Zellstoff umfasst.

15. Verfahren zur Herstellung der Schale (100) nach Anspruch 1, welches das Formen der Schale (100) aus pflanzlichem Zellstoff oder recyceltem pflanzlichem Zellstoff durch Spritzgießen von angefeuchtetem pflanzlichem Zellstoff oder recyceltem pflanzlichem Zellstoff umfasst.

## Revendications

1. Plateau (100) pour stocker des récipients de collecte de fluide biologique (44) comprenant :
un corps (102) comprenant :
une surface supérieure (112) comprenant une partie périmétrique (22) ayant un périmètre extérieur (26) et un périmètre intérieur (20) ; et
un évidement s'étendant vers le bas à partir du et entouré par le périmètre intérieur (20) de la partie périmétrique (22),
dans lequel l'évidement est divisé en une pluralité de puits (116a, 116b), chaque puits étant adapté pour recevoir au moins une partie d'un récipient de collecte de fluide biologique (44), et
une base (104) comprenant :
une extrémité inférieure fermée (105) ;
une extrémité supérieure ouverte (106) ; et
une paroi latérale (107) s'étendant entre celles-ci,
dans lequel l'extrémité inférieure fermée (105) et la paroi latérale (107) de la base (104) définissent un compartiment (109) qui reçoit le corps (102), et
**caractérisé en ce que**
l'extrémité inférieure fermée (105) de la base (104) comporte au moins un évidement (111) qui reçoit une extrémité inférieure (128a, 128b) de l'un de la pluralité de puits (116a, 116b) ; et
dans lequel le corps (102) et la base (104) du plateau (100) comprennent de la pâte végétale moulée.

2. Plateau (100) selon la revendication 1, dans lequel les parois latérales intérieures (34a, 34b) de la pluralité de puits (116a, 116b) adjacents à la partie périmétrique (22) sont définies par une paroi latérale intérieure de corps (18) s'étendant vers le bas à partir du périmètre intérieur (20) de la partie périmétrique (22), une cloison longitudinale (28) s'étendant dans une direction longitudinale (L) d'un premier côté (30) de la paroi latérale intérieure de corps (18) à un second côté opposé (32) de la paroi latérale intérieure de corps (18), et une pluralité de nervures (36a, 36b) s'étendant dans une direction transversale (T) entre la paroi latérale intérieure de corps (18) et la cloison (28).

3. Plateau (100) selon la revendication 1, dans lequel une paroi latérale intérieure de corps (18) s'étend vers le bas à partir du périmètre intérieur (20) de la partie périmétrique (22) et des parois latérales intérieures (34a, 34b) de la pluralité de puits (116a, 116b) qui ne sont pas adjacents à la partie périmétrique (22) sont définies par deux cloisons longitudinales (28) s'étendant dans une direction longitudinale (L) d'un premier côté (30) de la paroi latérale intérieure de corps (18) à un second côté (32) opposé de la paroi latérale intérieure de corps (18), et une pluralité de nervures (36a, 36b) s'étendant dans une direction transversale (T) entre les cloisons (28).

4. Plateau (100) selon la revendication 1, dans lequel la pluralité de puits (116a, 116b) ont une surface inférieure fermée (38a, 38b) et dans lequel de préférence au moins une partie de parois latérales intérieures (34a, 34b) de la pluralité de puits (116a, 116b) sont discontinues de sorte qu'une ouverture (40) est prévue entre des puits (116a, 116b) adjacents.

5. Plateau (100) selon la revendication 1, dans lequel une pluralité de puits (116a, 116b) sont définis par une paroi latérale intérieure de corps (18) s'étendant vers le bas à partir du périmètre extérieur (26) de la partie périmétrique (22), une cloison longitudinale (28) s'étendant dans une direction longitudinale (L) d'un premier côté (30) de la paroi latérale intérieure de corps (18) à un second côté (32) opposé de la paroi latérale intérieure de corps (18), et une pluralité de nervures (36a, 36b) s'étendant dans une direction transversale (T) entre la paroi latérale intérieure de corps (18) et la cloison (28) ou par deux cloisons longitudinales (28) s'étendant dans une direction longitudinale (L) à partir d'un premier côté (30) d'une paroi latérale intérieure de corps (18) s'étendant vers le bas du périmètre intérieur (20) de la partie périmétrique (22) à un second côté (32) opposé de la paroi latérale intérieure de corps (18), et une pluralité de nervures (36a, 36b) s'étendant dans une direction transversale (T) entre les cloisons (28), et l'ouverture (40) est définie par les nervures (36a, 36b).

6. Plateau (100) selon la revendication 1, dans lequel la pluralité de puits (116a, 116b) sont agencés en rangées (R1, R2, R3) s'étendant dans la direction longitudinale (L) du plateau (100).

7. Plateau (100) selon la revendication 6, dans lequel une position dans la direction longitudinale (L) d'extrémités d'une première rangée (R1) sont décalées d'une position dans une direction longitudinale (L) d'extrémités d'une deuxième rangée (R2) adjacentes dans la direction transversale (T) à un côté de la première rangée (R1) et/ou décalées d'une position dans la direction longitudinale (L) d'extrémités d'une troisième rangée (R3) adjacentes dans la direction transversale (T) à un côté opposé de la première rangée (R1).

8. Plateau (100) selon la revendication 1, dans lequel les parois latérales intérieures (34a, 34b) de la pluralité de puits (116a, 116b) ont une forme correspondant sensiblement à une forme du récipient de collecte de fluide biologique (44) qui doit être reçue par le plateau (100) et/ou dans lequel les surfaces inférieures fermées (38a, 38b) de la pluralité de puits (116a, 116b) ont une forme correspondant sensiblement à une forme d'une surface inférieure du récipient de collecte de fluide biologique (44) qui doit être reçue par le plateau (100) et/ou dans lequel la pluralité de puits (116a, 116b) sont adaptés pour contenir des récipients de collecte de fluide biologique (44) cylindriques.

9. Plateau (100) selon la revendication 1, comprenant en outre une paroi latérale extérieure (24) s'étendant vers le bas à partir du périmètre extérieur (26) de la partie périmétrique (22).

10. Plateau (100) selon la revendication 9, dans lequel la paroi latérale extérieure (24) est sensiblement perpendiculaire à la surface supérieure (112) ou dans lequel la paroi latérale extérieure (24) est inclinée par rapport à la surface supérieure (112) ou dans lequel la paroi latérale extérieure (24) comporte une pluralité de nervures (46).

11. Plateau (100) selon la revendication 10, dans lequel les nervures (46) sont estampées dans la paroi latérale extérieure (24) de sorte que les nervures (46) sont des évidements sur une surface extérieure de la paroi latérale extérieure (24) et des saillies sur une surface intérieure de la paroi latérale extérieure (24) ou les nervures (46) sont des saillies sur une surface extérieure de la paroi latérale extérieure (24) et des évidements sur une surface intérieure de la paroi latérale extérieure (24).

12. Plateau (100) selon la revendication 1, dans lequel la base (104) comprend en outre une bride (108) s'étendant latéralement vers l'extérieur à partir d'un périmètre supérieur de la paroi latérale (107) de la base (104), et au moins une partie de la partie périmétrique (22) du corps (102) est supportée par la bride (108) lorsque le corps (102) est reçu dans le compartiment (109) de la base (104).

13. Plateau (100) selon la revendication 1, dans lequel le plateau (100) comprend de la pâte végétale recyclée moulée.

14. Procédé pour fabriquer le plateau (100) selon la revendication 1, comprenant le moulage du plateau (100) à partir de pâte végétale ou de pâte végétale recyclée par thermoformage de pâte végétale ou de pâte végétale recyclée humidifiée.

15. Procédé pour fabriquer le plateau (100) selon la revendication 1, comprenant le moulage du plateau (100) à partir de pâte végétale ou de pâte végétale recyclée par moulage par injection de pâte végétale ou de pâte végétale recyclée humidifiée.
